# EUROPEAN PATENT APPLICATION

(11) **EP 3 020 438 A1**
(43) Date of publication of application: **18.05.2016**
(21) Application number: 14193109.7
(22) Date of filing: 13.11.2014
(51) Int. Cl.: A61M 16/00

(54) **Device for ventilating a patient and method for operating a device for ventilating a patient**

(71) Applicant: Linde AG, 80331 München (DE)
(72) Inventor: Jafri, Syed, London, W87HY (GB); Schmehl, Wolfgang, 82031 Grünwald (DE)
(74) Representative: Nordmeyer, Philipp Werner

(57) **Abstract**

The present invention pertains to a device (1) for ventilating a patient, including an invasive mechanical ventilator (2) for periodically providing a breathing gas to an invasive patient interface (20), wherein a gas injector (4) for injecting nitric oxide supplied by a source of nitric oxide (3) into the breathing gas supplied by the invasive mechanical ventilator (2) is provided.

## Description

### Technical Field

The invention relates to a device for ventilating a patient and a method for operating a device for ventilating a patient.

### Technological Background

Nitric oxide (NO) has many known biological functions. Ranging from neurotransmission, cellular differentiation to regulation of cellular oxygen consumption through effects on mitochondrial respiration, it also regulates the host immune response by e.g. inhibition of leukocyte adhesion and regulation of NFκB levels *in vivo.* Its use in treatment of diseases affecting the respiratory tract, however, bases on the relaxation of smooth muscle cells lining the vascular system. Hence, administration of NO leads to a reduction of local blood pressure, stimulates vasodilatation and thereby facilitates gas exchange in the alveoli of the lungs.

The high reactivity of NO in pure form causes limited solubility in aqueous solutions. Consequently, delivery of NO is typically performed by administration of a prodrug which is metabolically degraded, or through direct inhalation of gaseous NO (IgNO), diluted with an inert carrier gas.

Biologically produced NO is synthesized *in vivo* by both constitutive and inducible isozymes of the nitric oxide synthases (NOS), which catabolize L-arginine to NO and citrulline. Endothelial constitutive NOS (eNOS), present in the walls of bronchioles and pulmonary arterioles provide NO at nanomolar concentrations for regulating vessel tone. Isozymes of inducible NOS (iNOS) are present in many cell types; upon activation they temporarily produce NO at micromolar concentrations, an activity which, under pathological conditions, has been associated with production of superoxides, peroxynitrites, e.g. upon reperfusion injury of ischemic tissue, inflammation and cellular damage.

Endogenously induced NO oxidizes the iron atom of a haem moiety in the enzyme soluble guanylate cyclase (SGC) in the smooth muscle cells of the lower respiratory tract airways, in the pulmonary arteries and in the membranes of circulatory platelets, thereby activating the SGC. The activated SGC forms the second messenger cGMP, which in smooth muscle cells promotes calcium-dependent relaxation, causing vasodilation of blood vessels in the lower respiratory tract, thereby increasing blood flow through the pulmonary arteries and capillaries, and also dilation of the airways in the lower respiratory tract, thereby improving bulk gas transport into the alveoli and exchange of O₂ and CO₂. A further result is reduction of platelet aggregation on irregular surfaces (such as a constricted blood vessel) thereby lowering the probability of thrombosis (see WO 95 / 10315 A1).

Other functions of NO are as a neurotransmitter in the brain where it mediates the actions of the excitatory neurotransmitter glutamate in stimulating cGMP concentrations, and in the intestine where it promotes neuronal relaxation. NO also forms nitrosyl derivatives of tyrosine residues in certain functional proteins. However, tyrosine nitration, resulting from reaction of protein tyrosine residues with NO₂ or the peroxynitrite anion, is used as an indicator of cell damage, inflammation and NO production. In many disease states, oxidative stress increases the production of superoxide (•O₂⁻).

The toxicity of IgNO is associated with a variety of properties.
(a) Firstly, NO is swiftly absorbed by lung tissue and enters the blood stream, where it reacts very rapidly with haemoglobin, oxidizing the iron atom of one of the four haem moieties to the ferric form, thereby creating stable methaemoglobin (+ nitrite and nitrate ions). Methaemoglobin's three ferrous haem groups have far greater affinity for oxygen than the haemoglobin haem moieties, so that blood in which the proportion of methaemoglobin is elevated releases insufficient oxygen to the tissues.
(b) Secondly, in the presence of oxygen NO reacts rapidly to form nitrogen dioxide (NO₂), itself a toxic molecule and forming acidic compounds in aqueous environments. Gaseous NO₂ at 5 ppm is considered to be a toxic concentration, compared to standard administrations of IgNO at between 10 to 40, maximum up to 80 ppm. As lung disease frequently causes reduced respiratory function, patients are often administered an O₂-enriched air supply. In the presence of such an increased concentration of O₂ the probability of NO being oxidized to toxic NO₂ is correspondingly greater.
(c) Thirdly, NO reacts with superoxides to form toxic peroxynitrites, powerful oxidants capable of oxidizing lipoproteins and responsible, as are both NO and NO₂, for nitration of tyrosine residues. Peroxynitrite reacts nucleophilically with carbon dioxide, which is present at about 1 mM concentrations in physiological tissues, to form the nitrosoperoxycarbonate radical. This, in turn, degrades to form carbonate radical and NO₂, both of which are believed to be responsible for causing peroxynitrite-related cellular damage. Nitrotyrosine is used as an indicator of NO-dependent nitrative stress induced in many disease states, generally being absent or undetected in healthy subjects.

Since, in the presence of oxygen, the NO concentration determines the production rate of NO₂, over-delivery of NO will generate excessive quantities of toxic NO₂. Even if inhaled for only a short period, excess NO may form sufficient methaemoglobin to reduce oxygen delivery to the tissues to dangerously low levels, particularly in patients suffering from lung disease. Excess inert carrier gas accompanying administration of IgNO may deplete oxygen content of respiratory gas supply. On the other hand, under-administration of IgNO to patients requiring relaxation of the smooth muscles in pulmonary arteries may result in excessively high arterial blood pressures causing a low partial pressure of O₂ in alveolar blood (low P_{A}O₂). Consequently, precise control of the NO dosage is required at all times during administration of IgNO, in spite of irregular patient breathing patterns, fluctuations in ambient temperature and pressure, and depletion of the gas reservoir.

IgNO may be used to relax smooth muscle control of pulmonary arteriole diameter, for treating pulmonary hypertension in diseases such as acute respiratory distress syndrome (ARDS), in which impaired gas exchange and systemic release of inflammatory mediators ('acute phase proteins' and cytokines, particularly interleukins) cause fever and localised or systemic increases in blood pressure. IgNO will also relax smooth muscle control of bronchiole diameter, for treating emphysema in cases of ARDS and chronic obstructive pulmonary disease (COPD), in which the lower respiratory tract (particularly the lung parenchyma: alveoli and bronchioles) become inflamed. In COPD airways in the lower respiratory tract narrow and lung tissue breaks down, with associated loss of airflow and lung function which is not responsive to standard bronchodilating medication. IgNO administration may therefore assist in countering the 'pulmonary shunt', in which respiratory disease causes deregulation of the matching of the flow of air to the alveoli with the blood flow to the capillaries, which under normal conditions allows oxygen and carbon dioxide to diffuse evenly between blood and air (see WO 95 / 10315 A1).

IgNO is an effective microbicidal molecule and provides the advantage for treating infections of the respiratory tract that it acts directly *in situ,* whereas parenteral administration of drugs requires a high dosage to address systemic dilution and hepatic catabolism. Thus, NO has been shown to be an effective agent for killing *Mycobacterium tuberculosis* within cysts or tuberculi in a patient's lungs (see WO 00 / 30659 A1). IgNO may also be administered to treat pneumonia: pulmonary infection and inflammation (see WO 00 / 30659 A1). Pneumonia, which may accompany other respiratory or non-respiratory disease, is an inflammatory condition of the lung primarily affecting the alveoli resulting from infection with bacteria and/or viruses, less commonly by other organisms such as fungi or parasites. Bacteria generally enter the upper respiratory tract through aspiration of small quantities of microbial cells present in the nose or throat (particularly during sleep), or via airborne droplets. Systemic sepsis or septicaemia may also result in bacterial invasion of the lungs. Viral infection may occur through inhalation or distribution from the blood; in the lungs cells lining the airways, alveoli and parenchyma are damaged, and may render the patient more susceptible to bacterial infection of the respiratory tract. Response by the immune system to a respiratory tract infection may cause further damage through inflammation, particularly if the infection and the corresponding inflammation affect the lower respiratory tract. Macrophages and neutrophils located between pulmonary cells are mobilized to engulf and inactivate invading bacteria. The neutrophils also release cytokines, stimulating the immune response further. Fluid from surrounding blood vessels and from damaged cells, and containing defensive monocytes and invasive bacteria, flows into the alveoli in affected parts of the lung, thereby restricting influx of respiratory gas to the affected alveoli and reducing gas exchange efficiency, potentially causing a 'pulmonary shunt'.

In situations where patients require the application of an mechanical breathing apparatus, e.g. during surgical intervention with application of anesthetics, or e.g. when spontaneous breathing is not present, as in cases where patients reside in a comatose state, mechanical ventilation is applied. The principle of mechanical ventilation lies in the application of a positive air flow to a patient when the lung pressure is in equilibrium with ambient pressure, i.e. initiating and artificially performing an inhalation phase, and upon reaching a predefined threshold, allowing the patient to passively equalize the pressure towards ambient pressure, i.e. exhaling phase. The application of a positive air flow during the inhalation phase can be either autonomous, i.e. when the patient is incapable of spontaneous breathing, or supportive, when the patient is capable of initiating and/or partly performing the inhalation phase. The breathing interval is thereby artificially predefined.

Invasive mechanical ventilation is provided to intubated patients. Intubation of the patient is used for long term ventilation e.g. when the patient undergoes complicated surgical procedures or is in a comatose condition in an intensive care unit. The intubation is carried out by e.g. inserting an endotracheal tube to the upper airways of a patient, in order to provide a direct connection to the respiratory system. Use of such an intubation apparatus secures an open airway and prevents unwanted closure of the larynx and trachea by e.g. relaxation of the epiglottis. The intubation, however, not only potentially induces a foreign body reaction of the patients system leading to inflammation, but simultaneously increases the risk of unwanted accumulation or introduction of pathogens. Whereas current intubation apparatuses are produced of biocompatible materials, thereby drastically reducing the risk of a foreign body reaction, the risk of infection is not to be neglected.

Pathogens can be introduced either during intubation, can be derived from the respiratory system, can be aspirated by e.g. gastric reflux or are introduced during the process of mechanical ventilation. Although most designs eliminate or reduce the occurrence and/or establishment of dead spaces, the high humidity and body temperature form ideal growth conditions for e.g. bacteria to multiply. Both material and geometric properties furthermore provide an environment for these pathogens to colonize in a region that is difficult for the host's immune system to infiltrate or impairs induced extravasation, causing the readily formation of biofilms within 12 hours. Ineffective clearance of such a biofilm may cause further accumulation and eventually leads to droplet formation and spreading of the pathogen through the respiratory system.

The concomitant inflammation and infection of the respiratory system leads to pneumonia. Ventilator-associated pneumonia (VAP) is a life-threatening problem and the second leading cause of infection and death in hospital-acquired infections. According to Beth Augustyn: "Ventilator-Associated Pneumonia", Critical Care Nurse, Vol 27, No. 4 August 2007, 22.8 Percent of the patients receiving mechanical ventilation acquire VAP, which accounts for 86 percent of the patients acquiring hospital associated pneumonia. Not only does this increase the burden and morbidity of a patient, the required hospitalization of six days on average, costs of e.g. diagnostics, medical treatment and professional care together form severe health economics side effects of mechanical ventilation.

Reduced humidification, a reduced cough reflex and the impaired extravasation and infiltration of the immune system into the pathogenic area render the host's defense mechanism incapable of proper clearance. Prevention and treatment of these mechanical ventilation-related complications are therefore of key interest for increasing survival.

However, prevention and treatment are mostly inadequate. Whereas increased personal hygiene of trained medical professionals, along with e.g. increased oral hygiene of the patient, improved body positioning to reduce aspiration and even special coating of the intubation apparatus reduce the risk of developing VAP, the high incidence and prevalence show that these measurements are insufficient. In addition, administration of pharmaceuticals such as e.g. chlorhexidine, antibiotics and antimycotics increases the risk of resistance. Other methods that should prevent or reduce the risk of VAP or reduce the signs and symptoms thereof, such as frequent aspiration, prophylactic administration of antibiotics, saline lavage, re-intubation or frequent change of ventilation filters, in fact aggravate the patient's condition.

Endogenously produced NO is partially responsible for the cytotoxic actions of macrophages. The mechanisms discussed above relating to potential cell damaging activities of NO supplied either endogenously or exogenously, such as the production of superoxide, the nitration of tyrosine residues in critical proteins, and the stable binding to haem groups by NO to inhibit electron transport pathways and energy metabolism, are all mechanisms which will also apply to the activity of NO in countering infection. NO being an effective microbicidal molecule, IgNO offers the advantage for treating infections of the respiratory tract that it acts directly *in situ,* whereas parenteral administration of drugs requires a high dosage to address systemic dilution and hepatic catabolism.

Mechanical ventilation is known in the field as a treatment for inducing artificial breathing in a patient from CA 1108505 A1.

WO2009/057055A1 and WO2009/057056A2 discuss using nitric oxide to decontaminate the oropharyngeal area of an intubated mammal by administering the nitric oxide in an area between the proximal end and an inflatable balloon cuff of the catheter. The arrangement aims at avoiding inhalation of nitric oxide.

### Summary of the invention

It is an object of the present invention to provide a mechanism for the reduction of the occurrence of, or the prevention of ventilator-associated pneumonia.

In a first aspect, continuous use of gaseous nitric oxide as a prophylactic treatment for the prevention of ventilator-associated infections of the respiratory system caused by bacterial, viral, protozoal, fungal and/or microbial infections in a patient under invasive mechanical ventilation is suggested.

It is suggested to use nitric oxide for preventing ventilator associated pneumonia in an invasive mechanical ventilator.

Preferably, the nitric oxide is present in the breathing gas supplied to the patient by means of the invasive mechanical ventilator in a concentration of between 0.1 ppm and 200 ppm, preferably between 1 ppm and 160 ppm, more preferred between 1 ppm and 80 ppm, more preferred between 10 ppm and 40 ppm, more preferred between 15 ppm and 20 ppm. Accordingly, the concentrations of the nitric oxide may be very low but the continuous application thereof prevents or at least reduces the occurrence of ventilator associated pneumonia as the multiplication of bacteria, viruses and fungi can be slowed down or even supressed. When higher concentrations of the ranges given above are used, bacteria, viruses and fungi can be killed.

In order to achieve the best possible effect of the prevention, the nitric oxide is supplied to the patient continuously together with the breathing gas supplied by the invasive mechanical ventilator. When supplying the patient continuously with the nitric oxide it is preferred to use concentrations on the lower end of the above given ranges.

Invasive mechanical ventilation is understood to be an invasive ventilation method, in which the patient is intubated and its breathing is artificially regulated. Invasive mechanical ventilation is applied in situations in which the patient is incapable of spontaneous and/or autonomous breathing and is often required for a prolonged period of time.

The mucolytic and pulmonary blood pressure reducing properties of NO together with the microbicidal effect have an unexpected synergistic effect. While acute treatment to increase oxygen exchange in the alveoli uses high concentrations (i.e. up to maximal 80ppm) of NO, continuous long-term application with such levels is impossible due to associated high toxicity. With the present suggestion gaseous nitric oxide is used as a pharmaceutical treatment in a concentration of between 1 ppm and 40 ppm, preferably between 1 ppm and 25 ppm and more preferred between 1 ppm and 15 ppm, thereby allowing continuous and duration-independent treatment while effectively reducing ventilator-associated infections.

The use of such low doses is compliant with clinical levels and falls within the exposure levels for the approved indications. As is the case with neonatal infants, such doses can be applied for a prolonged time of >14 days, which exceeds the average time of onset of e.g. ventilator-associated pneumonia which lies between 48 and 96 hours for early onset and >96 hours for late onset. The continuous administration of such doses therefore allows an effective prevention of ventilator-associated infections of the respiratory system in patients.

In another aspect, a method of continuously providing an inhalable medicament by injecting nitric oxide in a concentration of between 1 ppm and 40 ppm, preferably between 1 ppm and 25 ppm and more preferred between 1 ppm and 15 ppm to the breathing gas of an invasive mechanical ventilator is suggested.

Preferably, the nitric oxide is injected into a flow of breathing gas which is provided under positive pressure by the invasive mechanical ventilator and the nitric oxide is injected together with a carrier gas such that the gas injected is already in the correct concentration.

If the concentration of the carrier gas is high, it might be necessary to inject additional oxygen into the flow of breathing gas in order to supply to the patient a breathable gas with a suitable concentration of oxygen.

To find a balance between the positive and negative characteristics of nitric oxide, the nitric oxide is preferably injected into the breathing gas of the invasive mechanical ventilator in a varying dose of between 1 ppm and 40 ppm, preferably between 1 ppm and 25 ppm and more preferred between 1 ppm and 15 ppm.

In another arrangement, the nitric oxide is supplied to the breathing gas in at least a pulse throughout an inhalation cycle, during every second breathing cycle or during every other natural number of breathing cycles except one. When supplying nitric oxide in pulses, higher concentrations may be used, in particular concentrations towards the upper end of the ranges given above.

To balance the negative effects of nitric oxide it is also contemplated to supply the nitric oxide to the breathing gas during an interval of between 1 minute to 60 minutes, preferably between 1 minute and 30 minutes, more preferably between 5 minutes and 20 minutes with an intermission between two subsequent intervals of between 1 minute to 1 day, preferably of between 1 hour and 8 hours, more preferably between 2 hours and 6 hours, most preferred between 3 hours and 5 hours. In other words, nitric oxide treatments are carried out intermittently and preferably with higher concentrations but in the intermissions the organism can recover again.

Furthermore, when using intervals with intermissions in between it is also possible to synchronize the intermissions with the growth rates of the bacteria, viruses and fungi to be suppressed. In other words, this administration regime aims at killing the bacteria, viruses and fungi at a certain point in time by administering a certain dosis of nitric oxide - either as a pulse or a number of subsequent pulses or as a continuous treatment during a treatment interval. Then the supply of nitric oxide is shut off. Remaining bacteria, viruses and fungi may then start multiplying again but are killed again before they have reached a critical concentration by administering the next interval or puls/pulses of nitric oxide. Accordingly, the duration of the intermission depends on the growth rate of the bacteria, viruses and fungi determined as critical.

In this respect it is to be understood that there is a correlation between the duration of the treatment interval and the concentration of the nitric oxide. The higher the concentration of the nitric oxide the shorter the duration of the treatment interval can be. For example, in order to suppress critical bacteria, viruses and fungi below a predetermined threshold, it might be necessary to supply nitric oxide in a concentration of 80ppm for 1 hour. As an alternative and in order to achieve the same result, a concentration of 40ppm for 2 hours could be administered. Accordingly, the negative effects of administering nitric oxide can be balanced with the duration of the treatment interval.

The objective given above is also solved by a device for ventilating a patient, including an invasive mechanical ventilator for periodically providing a breathing gas to an invasive patient interface. According to the invention a gas injector for injecting nitric oxide supplied by a source of nitric oxide into the breathing gas supplied by the invasive mechanical ventilator is provided.

By means of the injector it becomes possible to inject nitric oxide into the breathing gas of a patient such that the occurrence of ventilator associated pneumonia can be reduced or suppressed.

As a source of nitric oxide, nitric oxide can also be produced at the required location. Such production methods are known in the art. US 5,396,882 discloses e.g. the application of an electric arc to enrich NO from air, whereas US 2006/172018 provides a method for obtaining NO by controlling the diffusion and/or dissolution of nitride salts or other precursor compositions. NO can also be derived through a reaction with reduction agents. An example of such a method can e.g. be found in US 2011/220103. The methods described here should not be appreciated such that these are limiting, but merely provide examples from a plurality of alternative methods known in the art.

Preferably a controller programmed for controlling the gas injector for adjusting the injection of nitric oxide into the breathing gas to a predetermined concentration of nitric oxide is provided.

It is particularly preferred if the controller is programmed to control the gas injector to inject the nitric oxide such that a concentration of nitric oxide in the breathing gas of between 0.1 ppm and 200 ppm, preferably between 1 ppm and 160 ppm, more preferred between 1 ppm and 80 ppm, more preferred between 10 ppm and 40 ppm, more preferred between 15 ppm and 20 ppm is achieved. In other words, the controller controls the gas injector such that a preferred concentration of nitric oxide is present which is intended to reduce the occurrence of ventilator associated pneumonia but at the same time does not induce dangerous conditions in the patient if administered continuously and over a long period. Thus, in low concentrations, the nitric oxide may act as a "*continuous disinfection"* for the airways of the intubated patient. In higher concentrations the nitric oxide may act to disinfect the airways in in cycles.

Preferably, the controller is programmed to control the gas injector to inject the nitric oxide in a constant concentration to the breathing gas throughout the inhalation cycle or in at least a pulse in throughout an inhalation cycle, during every second breathing cycle or during every other natural number of breathing cycles except one.

The continuous application may also mean that the controller is programmed to control the gas injector to inject the nitric oxide every second breathing cycle or every other natural number of breathing cycles except one. Accordingly, the nitric oxide is added to the breathing gas not every breathing cycle, i.e. every inhalation phase, but every other or even less frequently. This application still enables the nitric oxide to prevent or reduce the occurrence of ventilator associated pneumonia.

It is also preferred to program the controller such that the gas injector injects the nitric oxide during an interval of between 1 minute to 60 minutes, preferably between 1 minute and 30 minutes, more preferably between 5 minutes and 20 minutes with an intermission between two subsequent intervals of between 1 minute to 1 day, preferably of between 1 hour and 8 hours, more preferably between 2 hours and 6 hours, most preferred between 3 hours and 5 hours. Accordingly, a treatment a day or any number of treatments a day might be carried out in order to reduce the occurrence of ventilator associated pneumonia.

Precise application of nitric oxide to the breathing gas can be achieved by the provision of a gas sensor for sensing the gas concentration of nitric oxide in the breathing gas administered to the patient interface wherein the gas sensor is in communication with the controller.

A method for operating a device for ventilating a patient, preferably a device as has been described before, including an invasive mechanical ventilator for periodically providing a breathing gas to an invasive patient interface is provided in order to achieve the object mentioned above. According to the invention, nitric oxide is injected into the breathing gas provided to the invasive patient interface.

The combination of the nitric oxide injector and the invasive mechanical ventilator enables the treatment of a patient with a positive air flow which is enriched with nitric oxide. The invasive mechanical ventilator senses the pressure in the airways and automatically induces a positive pressure and a positive airflow when a lower pressure threshold is achieved, thereby providing a positive airflow to the patient interface, and stops the provision of a positive airflow when an upper threshold is achieved, allowing passive efflux of air during a passive exhaling phase until the lower threshold is again achieved.

In order to meet the prescriptions, the controller is preferably programmed to control the injection of the nitric oxide into the breathing gas of the invasive mechanical ventilator during autonomous mechanical ventilation and during supportive mechanical ventilation during continuous mechanical ventilation and is duration-independent.

By the same token, it is preferred that the controller is programmed to interrupt the gas injector to provide nitric oxide when the invasive mechanical ventilator detects an exhaling phase.

To be in a position to analyse the gas in the patient interface, preferably a gas sensor for determining the concentration of nitric oxide, oxygen and/or nitrogen dioxide in the patient interface is present and the gas sensor is connected to the controller to adjust the concentration of nitric oxide injected.

The addition of gaseous nitric oxide during mechanical ventilation furthermore has an alleviating effect during infections of the lungs or upper airways by impairing multiplication of bacteria, viruses, protozoa, fungi, and/or microbes.

Depending on the desired distribution of the nitric oxide in the lung, the nitric oxide could also be injected in a single pulse or in multiple subsequent pulses such that different areas of the lung can be reached by the nitric oxide.

### Brief description of the drawings

The present disclosure will be more readily appreciated by reference to the following detailed description when being considered in connection with the accompanying drawings in which:
Figure 1 is a schematic view of a device for ventilating a patient in an intensive care unit;
Figure 2 is a schematic view of another device for ventilating a patient;
Figure 3 is a schematic diagram showing the breathing cycle in the device; and
Figure 4 is a schematic diagram showing the relation between concentration and duration of treatment.

### Detailed description of preferred embodiments

In the following, the invention will be explained in more detail with reference to the accompanying Figures. In the Figures, like elements are denoted by identical reference numerals and repeated description thereof may be omitted in order to avoid redundancies.

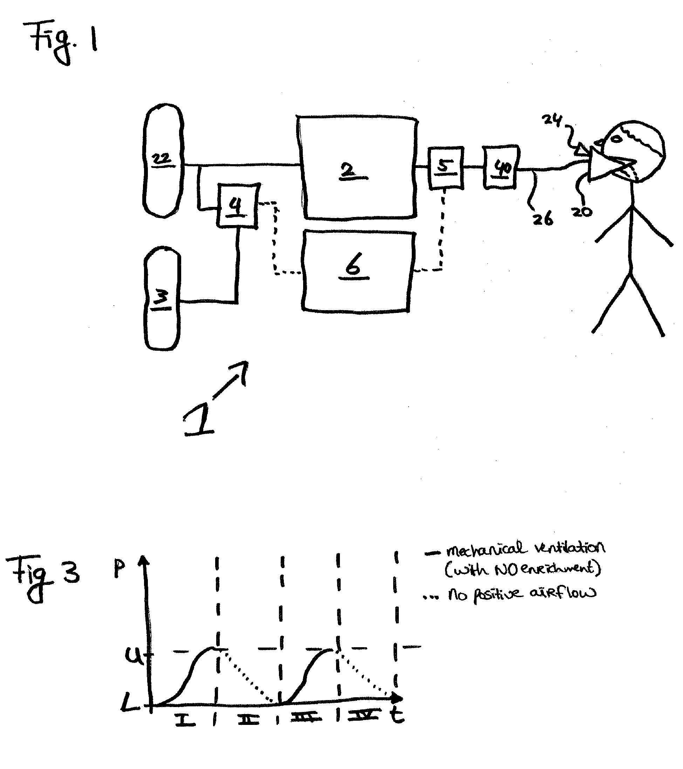

In Figure 1 a device 1 for ventilating a patient, in particular an invasive mechanical ventilator, is shown. Mechanical ventilators (or simply ventilators in this context) are machines designed to mechanically move breathable air into and out of the lungs of a patient to provide the mechanism of breathing for a patient who is physically either unable to breathe, or is breathing insufficiently.

In its simplest form, a modern invasive mechanical ventilator consists of a compressible air reservoir or turbine, air and oxygen supplies, a set of valves and tubes, and a disposable or reusable *"patient circuit*". The air reservoir is pneumatically compressed several times a minute to deliver room-air or in most cases an air/oxygen mixture to the patient. When overpressure is released, the patient will exhale passively due to the lungs' elasticity, the exhaled air being released usually through a one-way valve within the patient circuit called the patient manifold. The oxygen content of the inspired gas can be set from 21 percent (ambient air) to 100 percent (pure oxygen). Pressure and flow characteristics can be set mechanically or electronically.

The patient circuit usually consists of a set of three durable, yet lightweight plastic tubes, separated by function (e.g. inhaled air, patient pressure, exhaled air) wherein the patient-end of the circuit is invasive for the current description of the embodiment. The invasive method requires intubation, which for long-term ventilator dependence will normally be a tracheotomy cannula, as this is much more comfortable and practical for long-term care than is larynx or nasal intubation.

Ventilator-associated pneumonia (VAP) is a concomitant inflammation and infection of the respiratory system which leads to pneumonia. Ventilator-associated pneumonia (VAP) is a life-threatening condition and the second leading cause of infection and death in hospital-acquired infections. 22.8 Percent of the patients receiving mechanical ventilation acquire VAP, which accounts for 86 percent of the patients acquiring hospital associated pneumonia. Not only does this increase the burden and morbidity of a patient, the required hospitalization of six days on average, costs of e.g. diagnostics, medical treatment and professional care together form severe health economics side effects of invasive mechanical ventilation.

The device 1 includes an invasive mechanical ventilator 2 for providing a breathing gas under positive pressure and positive airflow to a patient who is physically unable to breath or is breathing insufficiently. Inhalation is provided periodically by means of the provision of the positive pressure and positive airflow to the patient. Exhalation can be provided by means of simply releasing the positive pressure provided and using the elastic properties of the lungs of the patients. Exhalation can also be assisted by applying a moderate negative pressure.

In the present example, the invasive mechanical ventilator 2 is shown as an intensive-care ventilator. The breathing gas is delivered via a hose 26 to an invasive patient interface 20 which is shown exemplary as an endotracheal tube. Generally, an intubation apparatus providing a direct access to the upper airways of the patient to be treated is used as the invasive patient interface 20.

In other embodiments, the invasive patient interface 20 may have any form which is suitable to supply a patient with a direct access to the upper airways, in particular when undergoing treatment in an intensive care unit. Accordingly, the patient interface 20 may be an endotracheal tube, a tracheostomy tube, a tracheal button, a catheter-based apparatus or any other known device to supply a direct access and a positive airflow to the upper airways of a patient.

The invasive mechanical ventilator 2 could also be provided in a different setup such as a transport ventilator, a neonatal ventilator or any other device which provides a positive airflow pressure to a patient in a ventilation setup in which the patient is incapable or insufficiently capable to breathe spontaneously.

The invasive mechanical ventilator 2 provides to the patient a breathing gas in the form of either ambient air, medical air or any other breathable gas mixture provided by a source of breathing gas 22 under positive pressure. The principle of the provision of a breathing gas to a patient under positive pressure in order to achieve in the patient a positive airflow is generally known.

The invasive mechanical ventilator 2 blows breathing gas at a prescribed positive pressure through the hose 26 to the invasive patient interface 20. In the gas path between the invasive mechanical ventilator 2 and the invasive patient interface 20 a positive pressure is build up and maintained. In the invasive patient interface 20, a pressure relief valve 24 is present which is provided in the form of a one-way valve, which is used for exhalation.

To prevent or reduce the occurrence of ventilator associated pneumonia (VAP), the following setup for injecting nitric oxide into the breathing air of the patient is provided: A source of gaseous nitric oxide 3 is provided which is intended to enrich the breathing gas which is to be supplied to the patient with nitric oxide in order to prevent the occurrence of VAP.

To this end, the source of gaseous nitric oxide 3 is connected to a gas injector 4 which is arranged for injecting nitric oxide provided by the source of gaseous nitric oxide 3 to the breathing gas. In the embodiment shown, the gas injector 4 injects the nitric oxide to the breathing gas upstream of the invasive mechanical ventilator 2. In a different embodiment, the gas injector 4 is arranged downstream of the invasive mechanical ventilator 2 or is integrated into the invasive mechanical ventilator 2.

In other words, the gas injector 4 can be situated between the source of breathing gas 22 and the invasive mechanical ventilator 2 or at the hose 26 close to the invasive mechanical ventilator 2 or close to the patient interface 20.

Additional means may be applied to perfuse nitric oxide enriched breathing air through proximal air canalization parts e.g. during the inhalation phase. In such an embodiment it is preferred that the gas injector 4 is situated close to the invasive patient interface 20 in order to be in a position to control the injection of the nitric oxide precisely.

A gas sensor 5 is present which can be situated in the invasive mechanical ventilator 2 or at any other position along the breathing gas path. The gas sensor 5 senses the gas concentrations of the breathing gas provided by invasive mechanical ventilator 2 and provides a feedback means for adjusting the injection of nitric oxide by the gas injector 4.

The gas sensor 5 may also be situated in a position different from the position shown in the Figures. In particular, said gas sensor 5 may be situated in the invasive patient interface 20.

When the patient exhales by means of the elasticity of the lungs, a pressure relief valve 24 or any other suitable exhaust opening situated in the invasive patient interface 20, releases the exhaled breathing gas of the patient to the outside. Accordingly, when the patient exhales, the invasive mechanical ventilator 2 does not provide a positive air flow of breathing gas towards the patient interface. The pressure relief valve 24 or any other suitable exhaust opening may be controlled by the invasive mechanical ventilator 2. The gas sensor 5 may be in communication with the invasive mechanical ventilator, such that the gas injector 4 is not controlled during an exhaling phase.

Accordingly, as schematically shown in Figure 3, the invasive mechanical ventilator 2 provides an air pressure between a lower threshold L and an upper threshold U periodically, depending on the setting of the control system of the invasive mechanical ventilator 2. The treatment plan of the patient may require a variation of the ventilation pattern such that the different breathing cycles might have a different shape. The invasive mechanical ventilator 2 may also take into account some reflexes of the patient when the patient attempts to breath spontaneously and may also take into account the exhalation pattern when determining the inhalation pattern. These applications and control methods are generally known.

However, in the simplest form of a treatment protocol, a breathing cycle is provided and the invasive mechanical ventilator 2 provides the breathing gas to the patient up to a pre-set upper pressure threshold U and starts again providing the breathing gas to the patient as soon as - after exhalation - a lower threshold L is reached. This is schematically shown during the inhalation phases I and III in Figure 3.

Upon reaching an air pressure above an upper threshold U, the invasive mechanical ventilator 2 no longer provides a positive gas flow from the invasive mechanical ventilator 2 towards the patient interface 20 and allows a passive exhalation in phases II and IV to take place. Again upon reaching an air pressure at a lower threshold L, the invasive mechanical ventilator 2 actively induces an inhaling phase III, continued by another exhaling phase IV as described above and this process is continuously performed.

A controller 6 is provided which is connected to the gas sensor 5 to receive the signal of the gas concentrations measured by gas sensor 5. The controller 6 is programmed for controlling the injection of the nitric oxide into the breathing gas by means of triggering the gas injector 4. The controller 6 may be in communication with the invasive mechanical ventilator 2. If the invasive mechanical ventilator 2 initiates an inhaling phase, the continuous injection of nitric oxide is triggered by the controller 6.

Accordingly, nitric oxide is only injected by the gas injector 4 into the breathing gas provided under positive airflow if the patient inhales. By this means nitric oxide consumption can be reduced. Furthermore, as nitric oxide is rapidly oxidized to nitrogen dioxide, the nitric oxide is preferably injected as late as possible into the airflow.

The nitric oxide is provided to the breathing gas by means of the gas injector 4 either continuously or intermittently. If the nitric oxide is provided continuously, the concentration of nitric oxide which is administered to the patient is constant during the breathing cycle. Accordingly, all regions of the lungs which are ventilated are exposed to nitric oxide in order to prevent, reduce or suppress the development of ventilator-associated pneumonia (VAP) and/or the concomitant inflammation and infection of the respiratory system leading to pneumonia.

However, nitric oxide can also be added to the breathing gas of the patient in a different pattern, for example every second breathing cycle only (or after any suitable number of breathing cycles). In order to treat different areas or regions within the lung of the patient, a single or a number of subsequent pulses P of nitric oxide can be injected into the positive flow of breathing gas detected by the flow rate sensor 5. By triggering the different spikes of the injection of nitric oxide, the depth within the lungs that can be reached by the nitric oxide can be varied. The different pulses may have differing widths, depending on the respective prescription.

In an alternative, the nitric oxide is injected into the breathing gas as a constant flow or varying flow of gas.

The controller 6 is programmed and arranged to control the gas injector 4 such that the concentration of the nitric oxide in the breathing gas can be controlled, preferably to a concentration of between 0.1 ppm and 200 ppm, preferably between 1 ppm and 160 ppm, more preferred between 1 ppm and 80 ppm, more preferred between 10 ppm and 40 ppm, more preferred between 15 ppm and 20 ppm.

Preferably, the controller 6 is further programmed for controlling the injection of the nitrous oxide via the gas injector 4 with a feedback loop provided by the gas sensor 5. If measured gas concentrations by gas sensor 5 do not match the predefined values, gas concentrations can be adjusted as such.

The concentration of the nitric oxide is chosen such that a reduction, suppression or prevention of multiplication and/or replication of bacteria, viruses, protozoae, fungi and/or microbes is achieved. Preferably, low concentrations are applied to reduce detrimental effects on host tissue, however, higher concentrations may be chosen if other effects such as e.g. vasodilatation, mucolytic properties and/or bactericidal effects are favorable for the patient and these concentrations can be applied continuously without reaching toxic *in vivo* levels.

The source of gaseous nitric oxide 3 preferably provides a carrier gas, for example N₂, such that the nitric oxide is provided in a concentration of about 100 ppm to 10000 ppm, preferably 1000ppm to 10000 ppm. The higher the concentration of the nitric oxide in the source of gaseous nitric oxide 3, the smaller a gas container for supplying the nitric oxide can be. This is preferred for distribution reasons.

As an alternative source of nitric oxide, nitric oxide can also be produced at the required location. Such production methods are known in the art. US 5,396,882 discloses e.g. the application of an electric arc to enrich NO from air, whereas US 2006/172018 provides a method for obtaining NO by controlling the diffusion and/or dissolution of nitride salts or other precursor compositions. NO can also be derived through a reaction with reduction agents. An example of such a method can e.g. be found in US 2011/220103. The methods described here should not be appreciated such that these are limiting, but merely provide examples from a plurality of alternative methods known in the art.

In order to make sure that the patient is provided with the desired concentration of nitric oxide in the breathing gas offered to the patient, the concentrated gas supplied by the source of gaseous nitric oxide 3 needs to be diluted. Furthermore, the increased N₂ content by the carrier gas in the gas supplied by the source of gaseous nitric oxide 3 needs to be supplemented by a suitable concentration of oxygen such that the patient does not suffer from low oxygen levels.

To provide a suitable mixture of gas which preferably comprises the oxygen content of ambient air, a supply of oxygen is provided which enables the addition of oxygen to the breathing gas which is offered under invasive mechanical ventilation to the patient.

However, the higher the concentration of nitric oxide in the source of gaseous nitric oxide 3, the lower the proportion of the carrier gas, for example nitrogen, such that it is no longer necessary to add oxygen in order to achieve a breathable gas. In other words, the carrier does not displace oxygen in a critical amount such that oxygen would not have to be added to the breathing gas for this reason alone.

In the invasive mechanical ventilator 2 the breathing curve of the patient is predefined and can be adjusted according to patient observation. Accordingly, the gas injector 4 is controlled by the controller 6 according to a predetermined scheme and preferable injects a mixture of nitric oxide and the carrier gas nitrogen upstream of a mixing channel 40 in which the gas is conducted in turbulent manner such as to ensure proper mixing.

The nitric oxide can be injected via the gas injector 4 just on the basis of the inhaled gas concentrations as determined by the gas sensor 5.

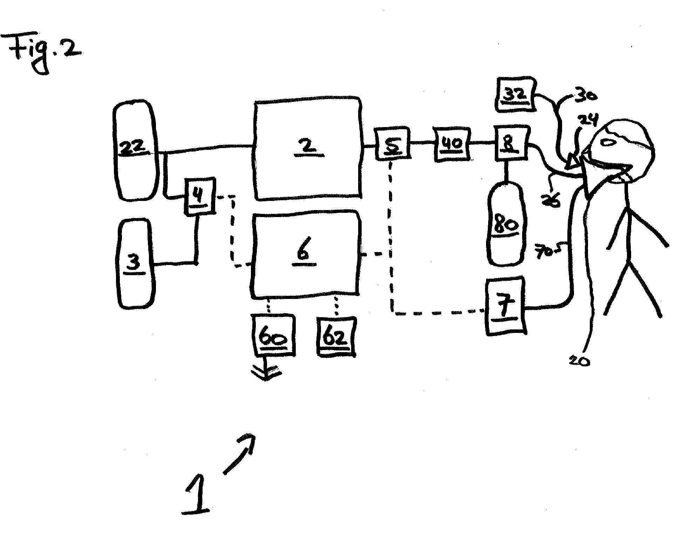

In Figure 2 another device 1 for the prevention of ventilator-associated infections of the respiratory system is shown which has, in principle, the same setup as the one shown in Figure 1. Additional preferred features of the device 1 are shown therein.

In a preferred embodiment, at the patient interface 20 a sample gas conduit 70 is provided which enables drawing test samples of air from the gas that is offered to the patient under positive airway pressure. The test samples can be analyzed in a gas sensor 7 such that the concentration of nitric oxide injected into the gas stream offered to the patient under positive airflow can be determined. The concentrations determined by the gas sensor 7 can be supplied to the controller 6 to further adjust the concentrations in order to ensure that the gas injector 4 injects the correct amount of nitric oxide such that the desired concentration is achieved at the patient interface 20 where the gas flows into the patient. The sample gas conduit 7 can be in communication or in-line with the pressure relief valve 24.

Furthermore, via the gas sensor 7 the occurrence of nitrogen dioxide in the invasive patient interface 20 can be monitored and if the concentration of nitrogen dioxide exceeds a predetermined level, the controller 6 lowers the concentration of nitric oxide injected into the gas flow, stops the injection of nitric oxide for one or more breathing cycles and/or triggers an alarm.

In a preferred embodiment, the gas sensor 7 also allows measuring the oxygen content of the gas offered to the patient under positive airway pressure upon invasive mechanical ventilation and sends feedback to the controller 6 such that the injection of additional oxygen supplied by an oxygen supply 80 via an oxygen injector 8 into the breathing gas of the patient can be adjusted accordingly. The oxygen concentration offered to the patient under positive airflow preferably resembles the oxygen concentration in ambient air, but can also be higher if the prescription calls for a higher concentration of oxygen.

The injection of oxygen into the flow of breathing gas takes place as close as possible to the patient interface 20 in order to reduce the contact of the oxygen with the nitric oxide to the least possible degree.

The controller 6 is preferably connected to a data communication module 60 as well as a monitoring module 62 which enables recording of the treatment data such that a doctor or control room in an intensive care unit can determine whether the patient was supplied with the prescribed doses of nitric oxide. The data communication module 60 can use any suitable form of communication with a doctor or control room such as, for example, wired data communication, wireless data communication or storage of data to a suitable data carrier. The format of the data communication preferably uses standard communication formats and protocols such as the internet protocol or any other telecommunication standards which enable communication of data between the device 1 and the doctor or control room.

Nitric oxide and nitrogen dioxide which is exhaled from the patient interface 20 can be treated before it is released to the environment. For the treatment of nitric oxide and nitrogen dioxide different methods and devices are available. However, in a regular setting, since the administered concentration of nitric oxide is below any toxic level, the exhaled gas from the patient interface 20 that is released into the surroundings does not require prior treatment because the overall concentrations of these gases are very low.

However, in case the gas exhaled by the patient as well as the gas withdrawn from the patient interface 20 is to be treated, the gases are collected via an output line 30 in a centralized waste gas treatment device 32 which treats the NO and nitrogen dioxide such that the treated gas can be released to the outside. In hospitals such devices for waste gas treatment are usually present.

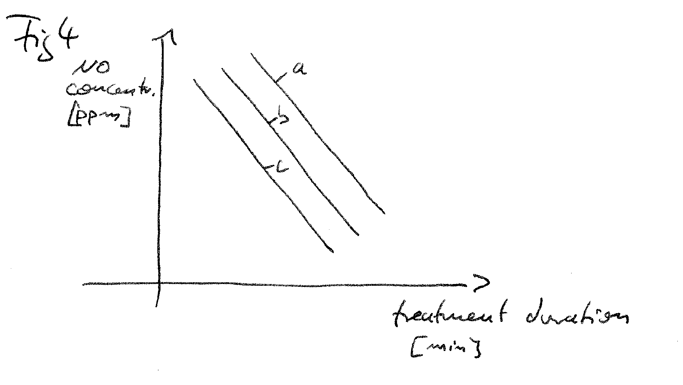

In Figure 4 a schematic diagram showing the interdependence between the concentration of NO supplied to the breathing gas of a patient and a treatment duration of a treatment interval. It can be seen that the treatment duration is longer when the concentration of NO is lower. However, when the concentration of NO increases, the treatment duration is shorter.

The graphs a, b, c show levels of different reduction of bacteria, viruses and fungi in a given setting. For example, graph a) shows a reduction of 90%, graph b) shows a reduction of 75% and graph c) shows a reduction of 50% of the bacteria, viruses and fungi in this setting. Accordingly, in order to achieve a higher reduction as shown with graph a, higher concentrations of NO would be necessary or longer treatment durations in a treatment interval.

Another parameter of a treatment regime would be the intermission between treatment intervals. This parameter, however, is not necessarily correlated with the concentrations and treatment durations of the administration of NO. It is rather determined on the basis of the growth rate of the bacteria, viruses and fungi considered harmful.

Starting from a situation after termination of a treatment interval, bacteria, viruses and fungi are at a low level. However, they start growing again such that they might approach a critical level again. Accordingly, before this critical level is reached the next treatment interval is to be initiated.

On the basis of these parameters, a treatment regime can be established which balances the positive and negative effects of NO and which helps achieve the goal of reducing or suppressing the occurrence of ventilator-induce pneumonia.

### List of reference numerals

- 1: device for ventilating a patient
- 2: invasive mechanical ventilator
- 20: invasive patient interface
- 22: source of breathing gas
- 24: pressure relief valve
- 26: hose
- 3: source of gaseous nitric oxide
- 30: output line
- 32: waste gas treatment
- 4: gas injector
- 40: mixing channel
- 5: gas sensor
- 6: controller
- 60: data communication module
- 62: monitoring module
- 7: gas sensor
- 70: sample gas conduit
- 8: oxygen injector
- 80: oxygen supply
- I: inhalation phase
- II: exhalation phase
- III: inhalation phase
- IV: exhalation phase
- L: lower threshold
- U: upper threshold
- p: pressure

## Claims

1. Nitric oxide for the use in preventing ventilator associated pneumonia in an invasive mechanical ventilator.

2. Nitric oxide according to claim 1, **characterized in that** the nitric oxide is present in the breathing gas supplied to the patient by means of the invasive mechanical ventilator in a concentration of between 0.1 ppm and 200 ppm, preferably between 1 ppm and 160 ppm, more preferred between 1 ppm and 80 ppm, more preferred between 10 ppm and 40 ppm, more preferred between 15 ppm and 20 ppm.

3. Nitric oxide according to claims 1 or 2, **characterized in that** the nitric oxide is supplied to the patient continuously together with the breathing gas supplied by the invasive mechanical ventilator.

4. Nitric oxide according to claims 1 or 2, **characterized in that** the nitric oxide is supplied in a constant concentration to the breathing gas throughout an inhalation cycle, in at least a pulse throughout an inhalation cycle, during every second breathing cycle or during every other natural number of breathing cycles except one.

5. Nitric oxide according to any of claims 1 to 4, **characterized in that** the nitric oxide is supplied to the breathing gas during an interval of between 1 minute to 60 minutes, preferably between 1 minute and 30 minutes, more preferably between 5 minutes and 20 minutes with an intermission between two subsequent intervals of between 1 minute to 1 day, preferably of between 1 hour and 8 hours, more preferably between 2 hours and 6 hours, most preferred between 3 hours and 5 hours.

6. Device (1) for ventilating a patient, including an invasive mechanical ventilator (2) for periodically providing a breathing gas to an invasive patient interface (20),
**characterized in that**
a gas injector (4) for injecting nitric oxide supplied by a source of nitric oxide (3) into the breathing gas supplied by the invasive mechanical ventilator (2) is provided.

7. The device (1) according to claim 6, **characterized by** a controller (6) programmed for controlling the gas injector (4) for adjusting the injection of nitric oxide into the breathing gas to a predetermined concentration of nitric oxide.

8. The device (1) according to claim 7, **characterized in that** the controller (6) is programmed to control the gas injector (4) to inject the nitric oxide such that a concentration of nitric oxide in the breathing gas of between 0.1 ppm and 200 ppm, preferably between 1 ppm and 160 ppm, more preferred between 1 ppm and 80 ppm, more preferred between 10 ppm and 40 ppm, more preferred between 15 ppm and 20 ppm is achieved.

9. The device (1) according to claim 6 or 7, **characterized in that** the controller (6) is programmed to control the gas injector (4) to inject the nitric oxide in a constant concentration to the breathing gas throughout the inhalation cycle, in at least a pulse throughout an inhalation cycle, during every second breathing cycle or during every other natural number of breathing cycles except one.

10. The device (1) according to any of claims 6 to 9, **characterized in that** the controller (6) is programmed to control the gas injector (4) to inject the nitric oxide during an interval of between 1 minute to 60 minutes, preferably between 1 minute and 30 minutes, more preferably between 5 minutes and 20 minutes with an intermission between two subsequent intervals of between 1 minute to 1 day, preferably of between 1 hour and 8 hours, more preferably between 2 hours and 6 hours, most preferred between 3 hours and 5 hours.

11. The device (1) according to any of claims 6 to 10, **characterized in that** it comprises a gas sensor (5) for sensing the gas concentration of nitric oxide in the breathing gas administered to the patient interface (20) wherein the gas sensor (5) is in communication with the controller (6).

12. Method for operating a device for ventilating a patient, preferably a device (1) according to any of claims 6 to 11, including an invasive mechanical ventilator (2) for periodically providing a breathing gas to an invasive patient interface (20),
**characterized in that**
nitric oxide is injected into the breathing gas provided to the invasive patient interface (20).

13. The method of claim 12, **characterized in that** nitric oxide is injected to achieve a concentration of between 0.1 ppm and 200 ppm, preferably between 1 ppm and 160 ppm, more preferred between 1 ppm and 80 ppm, more preferred between 10 ppm and 40 ppm, more preferred between 15 ppm and 20 ppm in the breathing gas provided to the invasive patient interface (20).

14. The method according to claim 12 or 13, **characterized in that** nitric oxide is injected in a constant concentration to the breathing gas throughout an inhalation cycle or in at least a pulse throughout an inhalation cycle.

15. The method according to any of claims 12 to 14, **characterized in that** the nitric oxide is injected during every second breathing cycle or every other natural number of breathing cycles except one.

16. The method according to any of claims 12 to 15, **characterized in that** the nitric oxide is injected to the breathing gas during an interval of between 1 minute to 60 minutes, preferably between 1 minute and 30 minutes, more preferably between 5 minutes and 20 minutes with an intermission between two subsequent intervals of between 1 minute to 1 day, preferably of between 1 hour and 8 hours, more preferably between 2 hours and 6 hours, most preferred between 3 hours and 5 hours.
